# EUROPEAN PATENT SPECIFICATION

(11) **EP 4 414 273 B1**
(45) Date of publication and mention of the grant of the patent: **22.04.2026**
(21) Application number: 24155362.7
(22) Date of filing: 01.02.2024
(51) Int. Cl.: B64G 1/60, B64G 1/46, A61F 5/451, A61F 5/455

(54) **UNIVERSAL COMMODE SYSTEM**
UNIVERSELLES COMMODE-SYSTEM
SYSTÈME COMMODE UNIVERSEL

(30) Priority: 07.02.2023 US 202363443831 P
(43) Date of publication of application: 14.08.2024
(73) Proprietor: Hamilton Sundstrand Corporation, Charlotte, NC 28217-4578 (US)
(72) Inventor: MESA, Jorge Ramon, League City, TX (US); KAUFMAN, Cory, St. Louis, MO (US); LITTON, Kevin, Houston, TX (US); EKRE, Bjorner, League City, TX (US); TADDEY, Edmund P., West Springfield, MA (US)
(74) Representative: Dehns

(56) References cited:
- US-A- 5 894 608
- US-A1- 2016 206 160
- US-A1- 2022 144 458
- "LIQUID/GAS SEPARATOR HANDLES VARYING LOADS", NTIS TECH NOTES, US DEPARTMENT OF COMMERCE. SPRINGFIELD, VA, US, 1 August 1992 (1992-08-01), pages 565, XP000325255, ISSN: 0889-8464
- PAYRA SYAMANTAK ET AL: "Artemis Removable-Canister Waste Accumulation System & Handler (ARC-WASH): A Portable Modular Space Lavatory", 2022 IEEE AEROSPACE CONFERENCE (AERO), IEEE, 5 March 2022 (2022-03-05), pages 1 - 12, XP034164327, DOI: 10.1109/AERO53065.2022.9843484

## Description

### CROSS-REFERENCE TO RELATED APPLICATIONS

This application claims the benefit of U.S. Provisional Application No. 63/443,831 filed February 7, 2023.

### BACKGROUND

The present disclosure relates to space toilets and, more particularly, to a universal commode system for space toilets.

The International Space Station (ISS) is the largest modular space station in low Earth orbit. The station serves as a microgravity and space environment research laboratory in which scientific research is conducted in astrobiology, astronomy, meteorology, physics and other fields. The ISS is suited for testing spacecraft systems and equipment required for possible future long-duration missions to the Moon and Mars. The ISS is further suited for habitation by humans for prolonged durations and involves the use of multiple life support systems.

Accordingly, there remains a need for improved life support systems to support human activity in the ISS, spacecraft and other space vehicles. US 2022/144458 A1 describes a space rated environmental control and life support system comprising two motors and an air/liquid separator. XP000325255 describes a liquid/gas separator. US 2016/206160 A1 describes a dosing pump. XP034164327 describes a removable-canister waste accumulation system. US 5 894 608 A describes a portable urine collection system.

### BRIEF DESCRIPTION

A universal commode system (UCS) is provided and includes an air/liquid separator, a pretreating assembly, a urine line connected to the air/liquid separator and to which the pretreating assembly is connected, a fan, a cabin air return line connected to the air/liquid separator and to the fan, a commode line connected to the cabin air return line, a first motor coupled to the air/liquid separator and configured to drive an operation of the air/liquid separator to separate air, liquid and pretreatment liquid flowing into the air/liquid separator from the urine line and from the pretreating assembly and a second motor independent of the first motor, coupled to the fan and configured to drive an operation of the fan to draw air out of the air/liquid separator and the commode line.

In accordance with additional embodiments, the pretreating assembly includes a pretreatment liquid source to provide a supply of the pretreatment liquid, a dose pump configured to pump a quantity of the pretreatment liquid from the pretreatment liquid source to the urine line and a pretreatment capping assembly interposed between the pretreatment liquid source and the dose pump.

In accordance with additional embodiments, the quantity of the pretreatment liquid pumped by the dose pump is in accordance with a current flow rate through the urine line.

In accordance with additional embodiments, the UCS further includes a urine funnel attached to a distal end of the urine line.

In accordance with additional embodiments, the UCS further includes a commode attached to a distal end of the commode line.

In accordance with additional embodiments, the UCS further includes an odor bacteria filter (OBF) disposed on the cabin air return line downstream from the fan.

In accordance with additional embodiments, the air/liquid separator comprises a drain and the UCS further includes a valve coupled to the drain and a temporary urine storage tank coupled to the valve.

In accordance with additional embodiments, the air/liquid separator includes a drain and the UCS further includes a valve coupled to the drain, an external temporary urine storage tank coupled to the valve and a capping assembly interposed between the valve and the external temporary urine storage tank.

Additional features and advantages are realized through the techniques of the present disclosure. Other embodiments and aspects of the disclosure are described in detail herein and are considered a part of the claimed technical concept. For a better understanding of the disclosure with the advantages and the features, refer to the description and to the drawings.

### BRIEF DESCRIPTION OF THE DRAWINGS

For a more complete understanding of this disclosure, reference is now made to the following brief description, taken in connection with the accompanying drawings and detailed description, wherein like reference numerals represent like parts:
FIG. 1 is a schematic diagram of a universal commode system (UCS) in accordance with an example which does not fall within the scope of the appended claims;
FIG. 2 is a schematic diagram of a universal commode system (UCS) with a reclamation configuration in accordance with embodiments; and
FIG. 3 is a schematic diagram of a processing system of the UCS of FIGS. 1 and 2 in accordance with embodiments.

### DETAILED DESCRIPTION

Space toilets are needed in order to facilitate human living in space. This is especially true for long term/duration stays in the ISS or other orbital vehicles and for trips leaving low Earth orbit. Currently, at the ISS, a universal waste management system (UWMS) is provided with conventional space toilets. The UWMS has been deemed excessively expensive, however, and presents certain technical issues that have yet to be overcome. Accordingly, there remains a need for simpler and cheaper UWMS solutions while still being able to fulfill the main toilet functions of being a medium for humans to urinate and defecate in a safe, practical and sanitary manner in a space environment.

As will be described below, a universal commode system (UCS) is provided with separate flow paths that each have an optimized commercial-off-the-shelf (COTS) motor so that one can handle gas effluent flows and the other can handle liquid effluent flows. This greatly simplifies the cost and complexity of the UCS along with minimizing power consumption (i.e., as compared to conventional commode systems that employs a gearbox and one motor). Also, a temporary urine storage tank is provided, in addition to the line that connects the liquid by-product to post processing external units, such as urine processors. This provides a novel level of operational flexibility for minimal urine storage. A minimum viable product (MVP) configuration presents urine pre-treat tablets (such as ones made out of oxone) inside of a urine hose as a simple and affordable method of urine treatment. The reclamation configuration adds a pre-treat assembly that includes a COTS pump and a pre-treat tank. A controller captures the pump's operational positional feedback to back calculate flow and thus dispense a corresponding right amount of pre-treat chemicals.

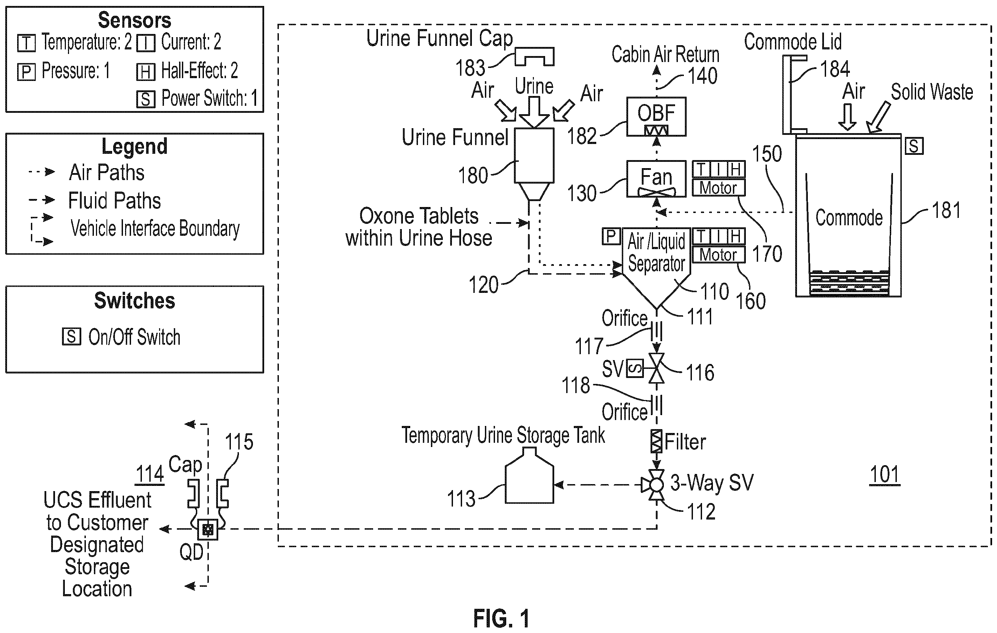

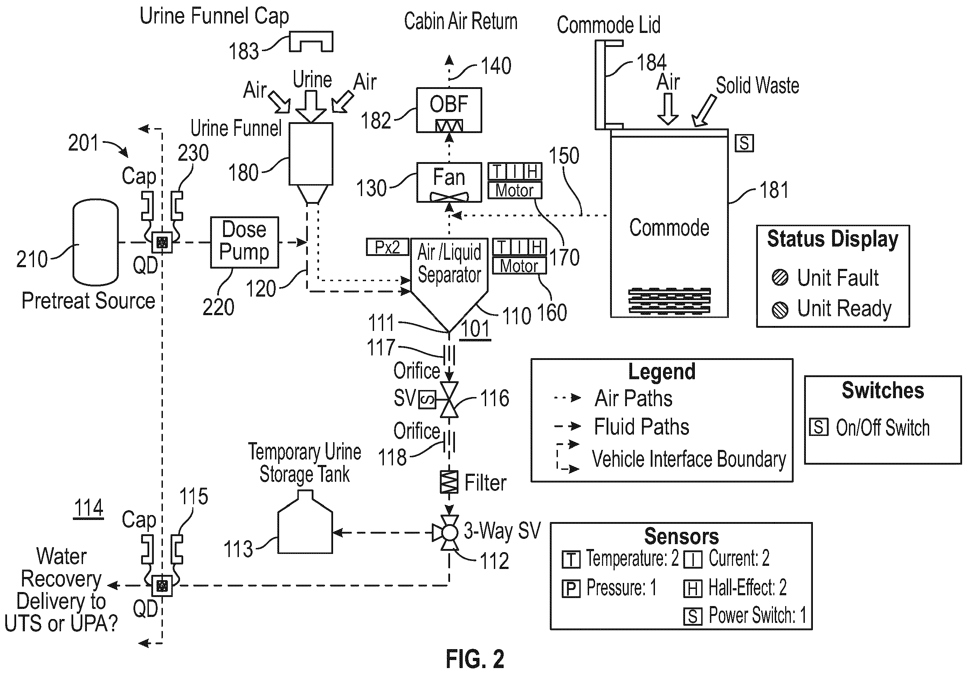

With reference to FIGS. 1 and 2, a UCS 101 is provided and includes an air/liquid separator 110 which can operate as a centrifugal air/liquid separator, a urine line 120 connected to an inlet of the air/liquid separator 110, a fan 130, a cabin air return line 140 connected to the air/liquid separator 110 and to the fan 130 and a commode line 150 connected to the cabin air return line 140. The UCS 101 further includes a first motor 160 and a second motor 170. The first motor 160 is coupled to the air/liquid separator 110 and is configured to drive an operation of the air/liquid separator 110 to separate air and liquid flowing into the air/liquid separator 110 from the urine line 120. The second motor 170 is independent of the first motor 160, coupled to the fan 130 and configured to drive an operation of the fan 130 to draw air out of the air/liquid separator 110 and the commode line 150.

As such, the UCS 101 provides for separated flow paths, one for mostly air and another for liquids. The air can be driven by the fan 130 and the liquids can be driven to flow by the operation of the air/liquid separator 110.

The UCS 101 also includes a urine funnel 180, a commode 181 and an odor bacteria filter (OBF) 182. The urine funnel 180 is attached to a distal end of the urine line 120 and includes a funnel cap 183. The commode 181 is attached to a distal end of the commode line 150 and includes a commode lid 184. The OBF 182 is disposed on the cabin air return line 140 downstream from the fan 130 to filter out odor causing bacteria from the cabin air return line 140 prior to air returning to a cabin.

The commode 181 can include or be provided with alternative fecal canister architectures including, but not limited to, soft canisters, canisters with varying geometries and/or materials, canisters with a hard shell assembly and/or canisters formed as small waste processors. In the latter case, the canister could utilize microporous bags, heat and partial pressure for instance to recover a sizable amount of water content from fecal matter and/or vomit, with some basic filtering and processing capabilities, which is then transferred for further processing and where canister heating could sterilize, treat and/or contain resulting solid waste. The commode 181 could also be provided with alternative commode bags to facilitate waste processing and water recovery from high-water content human waste that is currently generally untapped, such as vomit and feces. In these or other cases, a different fecal bag could be provided to be similar to those of trash compacting and processing systems (TCPS) where the bag may be microporous with dual layers (i.e., Teflon^{™} + silver or Nafion^{™} + Teflon^{™}).

In accordance with embodiments, the air/liquid separator 110 can include a drain 111 and the UCS 101 can further include a valve 112, such as a three-way valve, coupled to the drain 111 and at least one or both of a temporary urine storage tank 113 coupled to (i.e., one outlet of) the valve 112 and an external temporary urine storage tank 114 coupled to (i.e., one outlet of) the valve 112 as well as a capping assembly 115 interposed between the valve 112 and the external temporary urine storage tank 114. The UCS 101 can also include at least one additional valve 116 interposed between the drain 111 and the valve 112 as well as orifice openings 117, 118 on either side of the additional valve 116.

With the configurations described above, the UCS 101 can include or be provided with an adjustable/variable fan blower assembly. This will facilitate an interactive user interface (to be described below). In such an adjustable/variable fan blower assembly, the fan 130 could include or be provided as a variable speed fan blower and, in some cases, a variable solenoid valve essentially as an adjustable orifice so that the flowrate generated by the fan 130 can be adjusted. The user will thus get a range of (e.g., 2-3) levels of fan flow to choose from. How much suction occurs affects the smell, noise as well as the "draw" of the waste solids which could impact how many wipes are needed for cleaning. Still, the flow range to choose from will be well within operational bounds characterized as part of the development stage for the UCS 101. That is, the flow range to choose from will avoid excessive suction ("popcorning" where solid deposits occurs) or too little suction (where fecal matter does not migrate towards the bag enough driving usage of many wipes for cleaning).

In accordance with further embodiments, multiple sensors of varying types and switches can be deployed throughout the UCS 101 to enable its operation. These include temperature, current and Hall-effect sensors at each of the first motor 160 and the second motor 170, pressure sensors at the air/liquid separator 110 and an on/off switch at the commode 181.

As shown in FIG. 2, the UCS 101 can be provided with a reclamation configuration. In this case, the UCS 101 includes a pretreating assembly 201 that is connected to the urine line 120 downstream from the urine funnel 180 and the first motor 160 can be configured to drive the operation of the air/liquid separator 110 to separate air, liquid and pretreatment liquid flowing into the air/liquid separator 110 from the urine line 120 and from the pretreating assembly 201. The pretreating assembly 201 can be provided as a unitary feature and can include a pretreatment liquid source 210 to provide a supply of the pretreatment liquid, a dose pump 220 that is configured to pump a quantity of the pretreatment liquid from the pretreatment liquid source 210 to the urine line 120 and a pretreatment capping assembly 230 that is interposed between the pretreatment liquid source 210 and the dose pump 220. The quantity of the pretreatment liquid that is pumped by the dose pump 220 can be determined such that the quantity is in accordance with a current flow rate through the urine line 120.

In accordance with embodiments, operational positional feedback of the dose pump 220 can be used by a controller to back calculate a real-time flow along the urine line 120 and to thus dispense a corresponding amount or quantity of the pretreatment liquid (e.g., treated chemicals) into the urine line 120 while urination is occurring.

For the embodiments of FIGS. 1 and 2 described above, many or all of the features can be modularized so that overall structures/packaging of any one UCS is generally similar to another UCS. This allows for different customers to be provided with a system in line with their requirement and also facilitates future upgrades. In addition, the UCS 101 can include a customizable interactive user interface. In such cases, a tablet or touch screen can be provided to the user so that the user can customize some key settings to make their toilet and/or urination experience more amenable. The user will see the key perceived characteristics or variables that they desire to improve on including, but not limited to, noise, smell, cleaning, sound background, etc. The user would select a desired state or level for a variable or a set of variables and a controller would then, for instance, adjust an RPM setting of the fan 130 (i.e., the variable speed fan blower and/or the variable solenoid valve) so that the desired effect is achieved as much as possible yet in a safe manner. As noted above, the user will get levels of fan flows to choose from, so that they can tailor their experience and this could be saved (voluntary) as part of their profile so that the selections are remembered for the next time. In addition, a noise cancelling/dampening device can be provided and adjusted to minimize toilet operating noise levels whereas relaxing music, white noise, raining background, etc. could be output to facilitate or enhance the experience. The use of the tablet or touch screen could also inform as to when maintenance or replacement should occur (i.e., if several users are complaining about malodor, and even after fan settings adjustments, the issue is not being resolved, then the system should prompt appropriate personnel to take action by, e.g., changing the OBF 182, checking that the fan 130 and/or the air/liquid separator 110 are working properly, checking that seals on the commode 181 are working appropriately, etc.).

It is to be understood that the various features and embodiments of FIGS. 1 and 2 are generally interchangeable with one another and modularized for later modifications and updates.

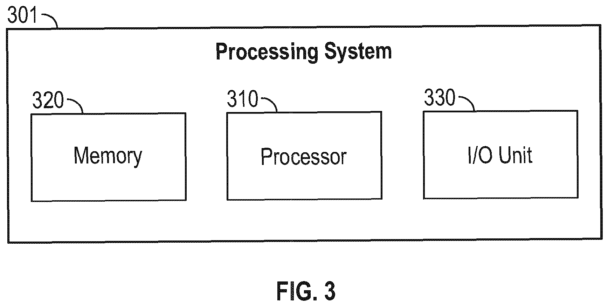

With reference to FIG. 3, the controller referred to above can include or be provided as a processing system 301. As shown in FIG. 3, the processing system 301 includes a processor 310, a memory 320 and an input/output (I/O) unit 330 by which the processor 310 is communicative with the multiple sensors, the switches and the controllable features described herein, such as, but not limited to, the valve 112, the additional valve 116, the first and second motors 160 and 170, the dose pump 220 and tablet or touch screen provided to the users). The memory 320 has executable instructions stored thereon, which are readable and executable by the processor 310. When the executable instructions are read and executed by the processor 310, the processor 310 is caused to operate generally as described herein.

Technical effects and benefits of the present disclosure are the provision of a UCS that offers separate flow paths that each have an optimized COTS motor so that one can handle gas effluent flows and the other can handle liquid effluent flows. This greatly simplifies the cost and complexity of the UCS along with minimizing power consumption. Also, a temporary urine storage tank is provided, in addition to the line that connects the liquid by-product to post processing external units, such as urine processors. This provides a novel level of operational flexibility for minimal urine storage. A minimum viable product (MVP) configuration presents urine pre-treat tablets inside of a urine hose as a simple and affordable method of urine treatment. The reclamation configuration adds a pre-treat assembly that includes a COTS pump and a pre-treat tank. A controller captures the pump's operational positional feedback to back calculate flow and thus dispense a corresponding right amount of pre-treat chemicals.

The description of the present disclosure has been presented for purposes of illustration and description, but is not intended to be exhaustive or limited to the technical concepts in the form disclosed. The embodiments were chosen and described in order to best explain the principles of the disclosure and the practical application, and to enable others of ordinary skill in the art to understand the disclosure for various embodiments with various modifications as are suited to the particular use contemplated.

## Claims

1. A universal commode system, UCS, comprising:
an air/liquid separator (110);
a pretreating assembly;
a urine line (120) connected to the air/liquid separator (110) and to which the pretreating assembly is connected;
a fan (130);
a cabin air return line (140) connected to the air/liquid separator (110) and to the fan (130);
a commode line (150) connected to the cabin air return line (140);
a first motor (160) coupled to the air/liquid separator (110) and configured to drive an operation of the air/liquid separator (110) to separate air, liquid and pretreatment liquid flowing into the air/liquid separator (110) from the urine line (120) and from the pretreating assembly; and
a second motor (170) independent of the first motor (160), coupled to the fan (130) and configured to drive an operation of the fan (130) to draw air out of the air/liquid separator (110) and the commode line (150).

2. The UCS according to claim 1, wherein the pretreating assembly comprises:
a pretreatment liquid source to provide a supply of the pretreatment liquid;
a dose pump configured to pump a quantity of the pretreatment liquid from the pretreatment liquid source to the urine line (120); and
a pretreatment capping assembly interposed between the pretreatment liquid source and the dose pump.

3. The UCS according to claim 2, wherein the quantity of the pretreatment liquid pumped by the dose pump is in accordance with a current flow rate through the urine line (120).

4. The UCS according to any preceding claim, further comprising a urine funnel (180) attached to a distal end of the urine line (120).

5. The UCS according to any preceding claim, further comprising a commode (181) attached to a distal end of the commode line (150).

6. The UCS according to any preceding claim, further comprising an odor bacteria filter, OBF (182), disposed on the cabin air return line (140) downstream from the fan (130).

7. The UCS according to any preceding claim, wherein the air/liquid separator (110) comprises a drain (111) and the UCS further comprises:
a valve (112) coupled to the drain (111); and
a temporary urine storage tank (113) coupled to the valve (112).

8. The UCS according to any of claims 1-6, wherein the air/liquid separator (110) comprises a drain (111) and the UCS further comprises:
a valve (112) coupled to the drain (111);
an external temporary urine storage tank (114) coupled to the valve (112); and
a capping assembly interposed between the valve (112) and the external temporary urine storage tank (114).

## Patentansprüche

1. Universelles Toilettensystem, UCS, umfassend:
einen Luft/Flüssigkeits-Separator (110);
eine Vorbehandlungsanordnung;
eine Urinleitung (120), die mit dem Luft/Flüssigkeits-Separator (110) verbunden ist und mit der die Vorbehandlungsanordnung verbunden ist;
ein Gebläse (130);
eine Kabinenluftrückführleitung (140), die mit dem Luft/Flüssigkeits-Separator (110) und mit dem Gebläse (130) verbunden ist;
eine Toilettenleitung (150), die mit der Kabinenluftrückführleitung (140) verbunden ist;
einen ersten Motor (160), der mit dem Luft/Flüssigkeits-Separator (110) gekoppelt ist und konfiguriert ist, um einen Betrieb des Luft/Flüssigkeits-Separators (110) anzutreiben, um Luft, Flüssigkeit und Vorbehandlungsflüssigkeit, die von der Urinleitung (120) und von der Vorbehandlungsanordnung in den Luft/Flüssigkeits-Separator (110) strömen, zu trennen; und
einen zweiten Motor (170), der von dem ersten Motor (160) unabhängig ist, mit dem Gebläse (130) gekoppelt ist und konfiguriert ist, um einen Betrieb des Gebläses (130) anzutreiben, um Luft aus dem Luft/Flüssigkeits-Separator (110) und der Toilettenleitung (150) abzusaugen.

2. UCS nach Anspruch 1, wobei die Vorbehandlungsanordnung umfasst:
eine Vorbehandlungsflüssigkeitsquelle, um einen Vorrat der Vorbehandlungsflüssigkeit bereitzustellen;
eine Dosierpumpe, die konfiguriert ist, um eine Menge der Vorbehandlungsflüssigkeit von der Vorbehandlungsflüssigkeitsquelle zu der Urinleitung (120) zu pumpen; und
eine Vorbehandlungsdeckelanordnung, die zwischen der Vorbehandlungsflüssigkeitsquelle und der Dosierpumpe angeordnet ist.

3. UCS nach Anspruch 2, wobei die Menge der Vorbehandlungsflüssigkeit, die durch die Dosierpumpe gepumpt wird, in Übereinstimmung mit einer aktuellen Durchflussrate durch die Urinleitung (120) steht.

4. UCS nach einem der vorhergehenden Ansprüche, ferner umfassend einen Urintrichter (180), der an einem distalen Ende der Urinleitung (120) angebracht ist.

5. UCS nach einem der vorhergehenden Ansprüche, ferner umfassend eine Toilette (181), die an einem distalen Ende der Toilettenleitung (150) angebracht ist.

6. UCS nach einem der vorhergehenden Ansprüche, ferner umfassend einen Geruchsbakterienfilter(OBF) (182), der an der Kabinenluftrückführleitung (140) stromabwärts von dem Gebläse (130) angeordnet ist.

7. UCS nach einem der vorhergehenden Ansprüche, wobei der Luft/Flüssigkeits-Separator (110) einen Ablauf (111) umfasst und das UCS ferner umfasst:
ein Ventil (112), das mit dem Ablauf (111) gekoppelt ist; und
einen temporären Urinspeichertank (113), der mit dem Ventil (112) gekoppelt ist.

8. UCS nach einem der Ansprüche 1-6, wobei der Luft/Flüssigkeits-Separator (110) einen Ablauf (111) umfasst und das UCS ferner umfasst:
ein Ventil (112), das mit dem Ablauf (111) gekoppelt ist;
einen externen temporären Urinspeichertank (114), der mit dem Ventil (112) gekoppelt ist; und
eine Deckelanordnung, die zwischen dem Ventil (112) und dem externen temporären Urinspeichertank (114) angeordnet ist.

## Revendications

1. Système de toilettes universel, UCS, comprenant :
un séparateur air/liquide (110) ;
un ensemble de prétraitement ;
une conduite d'urine (120) reliée au séparateur air/liquide (110) et à laquelle est relié l'ensemble de prétraitement ;
un ventilateur (130) ;
une conduite de retour d'air de cabine (140) reliée au séparateur air/liquide (110) et au ventilateur (130) ;
une conduite de toilettes (150) reliée à la conduite de retour d'air de cabine (140) ;
un premier moteur (160) couplé au séparateur air/liquide (110) et configuré pour entraîner le fonctionnement du séparateur air/liquide (110) afin de séparer l'air, le liquide et le liquide de prétraitement s'écoulant dans le séparateur air/liquide (110) à partir de la conduite d'urine (120) et de l'ensemble de prétraitement ; et
un second moteur (170) indépendant du premier moteur (160), couplé au ventilateur (130) et configuré pour entraîner le fonctionnement du ventilateur (130) afin d'extraire l'air du séparateur air/liquide (110) et de la conduite de toilettes (150).

2. UCS selon la revendication 1, dans lequel l'ensemble de prétraitement comprend :
une source de liquide de prétraitement permettant de fournir un approvisionnement en liquide de prétraitement ;
une pompe doseuse configurée pour pomper une quantité du liquide de prétraitement de la source de liquide de prétraitement vers la conduite d'urine (120) ; et
un ensemble d'obturation de prétraitement intercalé entre la source de liquide de prétraitement et la pompe doseuse.

3. UCS selon la revendication 2, dans lequel la quantité du liquide de prétraitement pompée par la pompe doseuse est conforme à un débit de courant à travers la conduite d'urine (120).

4. UCS selon une quelconque revendication précédente, comprenant en outre un entonnoir à urine (180) fixé à une extrémité distale de la conduite d'urine (120).

5. UCS selon une quelconque revendication précédente, comprenant en outre des toilettes (181) raccordées à une extrémité distale de la conduite de toilettes (150).

6. UCS selon une quelconque revendication précédente, comprenant en outre un filtre à bactéries odorantes, OBF (182), disposé sur la conduite de retour d'air de cabine (140) en aval du ventilateur (130).

7. UCS selon une quelconque revendication précédente, dans lequel le séparateur air/liquide (110) comprend une vidange (111) et l'UCS comprend en outre :
une vanne (112) accouplée à la vidange (111) ; et
un réservoir de stockage temporaire d'urine (113) accouplé à la vanne (112).

8. UCS selon l'une quelconque des revendications 1 à 6, dans lequel le séparateur air/liquide (110) comprend une vidange (111) et l'UCS comprend en outre :
une vanne (112) accouplée à la vidange (111) ;
un réservoir externe de stockage temporaire d'urine (114) accouplé à la vanne (112) ; et
un ensemble d'obturation intercalé entre la vanne (112) et le réservoir externe de stockage temporaire d'urine (114).
